(19) Europäisches Patentamt

European Patent Office

Office européen des brevets

(11) Veröffentlichungsnummer: **0 512 035 B1**

(12) **EUROPÄISCHE PATENTSCHRIFT**

(45) Veröffentlichungstag der Patentschrift: **30.11.94**

(21) Anmeldenummer: **91903669.9**

(22) Anmeldetag: **18.01.91**

(86) Internationale Anmeldenummer:
**PCT/EP91/00084**

(87) Internationale Veröffentlichungsnummer:
**WO 91/11424 (08.08.91 91/18)**

(51) Int. Cl.5: **C07C 69/708**, D21C 5/02,
C07C 67/30

(54) **ALKOXYLIERTE VERBINDUNGEN, HERGESTELLT AUS EPOXIDIERTEN CARBONSÄUREDERIVATEN.**

(30) Priorität: **26.01.90 DE 4002213**

(43) Veröffentlichungstag der Anmeldung:
**11.11.92 Patentblatt 92/46**

(45) Bekanntmachung des Hinweises auf die
Patenterteilung:
**30.11.94 Patentblatt 94/48**

(84) Benannte Vertragsstaaten:
**AT CH DE FR GB LI SE**

(56) Entgegenhaltungen:
**EP-A- 0 067 333
DE-A- 3 118 192
DE-A- 3 318 596
DE-A- 3 839 479
US-A- 3 663 583**

(73) Patentinhaber: **Henkel Kommanditgesellschaft auf Aktien**

**D-40191 Düsseldorf (DE)**

(72) Erfinder: **UPHUES, Günter
Robert-Koch-Strasse 45
D-4019 Monheim (DE)**
Erfinder: **PLOOG, Uwe, Dr.
Haydnweg 6
D-5657 Haan (DE)**
Erfinder: **DAUTE, Peter, Dr.
Kreuzeskirchstrasse 5
D-4300 Essen 1 (DE)**
Erfinder: **STOLL, Gerhard, Dr.
Danziger Strasse 69
D-4052 Korschenbroich 1 (DE)**
Erfinder: **SCHRECK, Berthold, Dr.
Mauerstrasse 26
D-4000 Düsseldorf (DE)**

**Beschreibung**

Die Erfindung betrifft alkoxylierte Verbindungen von epoxidierten $C_{10-22}$-Carbonsäurederivaten, ein Verfahren zur Herstellung sowie die Verwendung dieser alkoxylierten Verbindungen.

Ethoxylierte und/oder propoxylierte Ricinusöle werden beispielsweise in kosmetischen Zubereitungen, Waschmitteln oder Schmierölen oder als Antistatica für Nylonteppiche eingesetzt (Kirk-Othmer: "Encyclopedia of Chemical Technology", Band 5, Seite 9, John Wiley New York (1979)). Bei Verwendung ethoxylierter und/oder propoxylierter Rizinusöle muß jedoch in Kauf genommen werden, daß die auf dem Markt erhältlichen Mengen an Rizinusöl und damit auch an ethoxylierten und/oder propoxylierten Rizinusölen starken Schwankungen unterworfen sind. Mißernten in den Hauptanbaugebieten Brasilien und Indien führen in mehr oder weniger großen Abständen zu einer Verknappung des Ausgangsmaterials Rizinusöl. Es besteht daher ein Bedürfnis nach einem gleichwertigen Ersatz für alkoxylierte Rizinusöle. Vor allem sollte das Austauschprodukt von einer breiteren, weniger krisenanfälligen Rohstoffbasis aus zugänglich sowie ökologisch und toxikologisch unbedenklich sein.

EP-A-67 333 offenbart ein Verfahren zum Deinken von bedrucktem Altpapier durch Behandlung des Altpapiers im Stofflöser bei alkalischen pH-Werten mittels Alkalisilikat und oxidativ wirkenden Bleichmitteln. Bei diesen Verfahren werden nichtionische Dispergatoren wie Fettsäurepolyglykolester oder ethoxylierte Fettsäuremono- und/oder diglyceride eingesetzt.

Es wurde nun gefunden, daß epoxiderte $C_{10-22}$-Carbonsäurederivate, die mit $C_{2-4}$-Alkylenoxiden in Gegenwart von Alkoxylierungskatalysatoren sowie ein- und/oder mehrwertigen Alkoholen umgesetzt wurden, als Ersatz für alkoxylierte Rizinusöle eingesetzt werden können.

Erfindungsgegenstand sind dementsprechend alkoxylierte Verbindungen, hergestellt durch Umsetzung von epoxidierten $C_{10-22}$-Carbonsäurederivaten mit $C_{2-4}$-Alkylenoxiden in Gegenwart von Alkoxylierungskatalysatoren sowie ein- und/oder mehrwertigen Alkoholen.

Weiterer Erfindungsgegenstand ist ein Verfahren zur Herstellung von alkoxylierten Verbindungen, welches dadurch gekennzeichnet ist, daß epoxidierte $C_{10-22}$-Carbonsäurederivate mit $C_{2-4}$-Alkylenoxiden in Gegenwart von Alkoxylierungskatalysatoren sowie ein- und/oder mehrwertigen Alkoholen bei Temperaturen zwischen 110 und 220 °C und Drücken zwischen $10^5$ und $2 \cdot 10^6$ Pa umgesetzt werden.

Die erfindungsgemäßen alkoxylierten Verbindungen werden nach gängigen organischen Synthesemethoden hergestellt, indem epoxidierte $C_{10-22}$-Carbonsäurederivate mit $C_{2-4}$-Alkylenoxiden in Gegenwart von Alkoxylierungskatalysatoren, beispielsweise Natriummethylat und/oder Kaliumhydroxid, sowie ein- und/oder mehrwertigen Alkoholen bei Temperaturen vorzugsweise zwischen 150 und 190 °C und Drücken vorzugsweise zwischen $3 \cdot 10^5$ und $9 \cdot 10^5$ Pa umgesetzt werden. Die Alkylenoxide werden in solchen Mengen eingesetzt, daß der Alkylenoxidgehalt der erhaltenen alkoxylierten Verbindungen vorzugsweise zwischen 20 und 90 Gew.-%, besonders bevorzugt zwischen 40 und 80 Gew.-% liegt. Ethylenoxid und/oder Propylenoxid werden als $C_{2-4}$-Alkylenoxide bevorzugt. Sofern Ethylenoxid und Propylenoxid eingesetzt werden, können die beiden Alkylenoxide gleichzeitig oder nacheinander der Reaktionsmischung zugesetzt werden. Die ein- und/oder mehrwertigen Alkohole werden in Mengen vorzugsweise von 0,1 bis 2,0, besonders bevorzugt von 0,2 bis 1,0 OH-Gruppen, bezogen auf eine Epoxid-Gruppe, eingesetzt. Als ein- und/oder mehrwertige Alkohole eignen sich lineare und/oder verzweigtkettige, gegebenenfalls mit 1 bis 30 Mol $C_{2-4}$-Alkylenoxid-Einheiten alkoxylierte $C_{1-18}$-Alkylalkohole, beispielsweise Methanol, Ethanol und/oder Stearylalkohol, lineare und/oder verzweigtkettige, gegebenenfalls mit 1 bis 30 Mol $C_{2-4}$-Alkylenoxid-Einheiten alkoxylierte Alkandiole, beispielsweise Ethylenglycol, 1,2-Propandiol, 1,3-Propandiol, 1,4-Butandiol, 1,6-Hexandiol und/oder 1,12-Dodecandiol, Glycerin, Diglycerin, Polyglycerin, Trimethylolpropan, Pentaerytrit und/oder Zuckeralkohole, wie Mannit und/oder Sorbit.

Die als Edukte für die Herstellung der erfindungsgemäßen alkoxylierten Verbindungen zum Einsatz gelangenden epoxidierten $C_{10-22}$-Carbonsäurederivate sind durch Epoxidierung von ungesättigten $C_{10-22}$-Carbonsäurederivaten zugänglich. Gemäß dem in DE-PS 857 364 beschriebenen Verfahren können ungesättigte Carbonsäurederivate durch Umsetzung mit Peressigsäure in Anwesenheit saurer Katalysatoren oder mit in situ aus Ameisensäure und Wasserstoffperoxid gebildeter Perameisensäure epoxidiert werden. Die Jodzahlen der erhaltenen Epoxidierungsprodukte liegen unterhalb 20, vorzugsweise unterhalb 15. Als ungesättigte Carbonsäurederivate eignen sich alle OH-gruppenfreien, natürlich vorkommenden und/oder synthetisch herstellbaren Carbonsäurederivate, die Carbonsäurereste mit wenigstens einer oder zwei Doppelbindungen in 9-und/oder 13-Stellung besitzen, beispielsweise 9c-Dodecensäure-, 9c-Tetradecensäure-, 9c-Hexadecensäure-, 9c-Octadecensäure-, 9t-Octadecensäure-, 9c,12c-Octadecadiensäure-, 9c,12c,15c-Octadecatriensäure-, 9c-Eicosensäure- und/oder 13c-Docosensäurederivate und/oder Mischungen mit wenigstens einem hohen Gehalt solcher ungesättigten Carbonsäurederivate. Ungesättigte Carbonsäurederivate, die $C_{16-22}$-Carbonsäurereste mit wenigstens ein oder zwei Doppelbindungen in 9- und/oder

2

13-Stellung enthalten, werden bevorzugt. Geeignete ungesättigte Carbonsäurederivate sind beispielsweise ungesättigte $C_{10-22}$-Carbonsäureester, ungesättigte $C_{10-22}$-Carbonsäureamide, ungesättigte $C_{10-22}$-Carbonsäuremono-und/oder -di$C_{1-4}$-alkylamide und/oder ungesättigte $C_{10-22}$-Carbonsäuremono- und/oder -di-$C_{1-4}$-alkanolamide. Ungesättigte $C_{10-22}$-Carbonsäurealkylester mit 1 bis 18 C-Atomen im einwertigen Alkoholrest und/oder Mono-, Di- und/oder Triglyceride, die $C_{10-22}$-Carbonsäurereste mit wenigstens einer oder zwei Doppelbindungen in 9- und/oder 13-Stellung enthalten, werden bevorzugt.

Beispiele für ungesättigte $C_{10-22}$-Carbonsäure-$C_{1-18}$-alkylester, die in an sich bekannter Weise durch Veresterung der entsprechenden ungesättigten Carbonsäuren oder durch Umesterung der entsprechenden Mono-, Di- und/oder Triglyceride mit $C_{1-18}$-Alkylalkoholen, beispielsweise Methanol, Ethanol, Propanal, Butanol, Isobutanol, 2-Ethylhexanol, Decanol und/oder Stearylalkohol, zugänglich sind, sind Palmitoleinsäuremethylester, Ölsäuremethylester, Ölsäureethylester, Ölsäureisobutylester, Ölsäure-2-ethylhexylester und/oder Ölsäuredecylester und/oder $C_{10-22}$-Carbonsäure-$C_{1-18}$-alkylestergemische mit wenigstens einem hohen Gehalt an solchen $C_{10-22}$-Carbonsäure-$C_{1-18}$-alkylestern, die in den Carbonsäureresten wenigstens eine oder zwei Doppelbindungen in 9- und/oder 13-Stellung haben, wie Palmfettsäuremethylester, Sojafettsäuremethylester, Sojafettsäure-2-ethylhexylester, Rübfettsäuremethylester und/oder Talgfettsäureethylester. Als Mono-, Di- und/oder Triglyceride, die OH-gruppenfreie, ungesättigte $C_{10-22}$-Carbonsäurereste mit wenigstens einer oder zwei Doppelbindungen in 9-und/oder 13-Stellung enthalten, eignen sich insbesondere Fette und/oder Öle natürlichen Ursprungs, deren Carbonsäuregehalt sich überwiegend aus ungesättigten $C_{10-22}$-Carbonsäuren mit wenigstens einer oder zwei Doppelbindungen in 9- und/oder 13-Stellung, vorzugsweise überwiegend aus ungesättigten $C_{16-22}$-Carbonsäuren mit wenigstens einer oder zwei Doppelbindungen in 9- und/oder 13-Stellung zusammensetzt, wie Olivenöl, Leinöl, Sonnenblumenöl, Safloröl, Sojaöl, Erdnußöl, Baumwollsaatöl, erucasäurereiches und/oder erucasäurearmes Rüböl, Palmöl, Schmalz und/oder Talg.

Die erfindungsgemäßen alkoxylierten Verbindungen eignen sich zur Entfernung von Druckfarben aus bedruckten Altpapieren und/oder Papierkreislaufwässern. Weiterer Erfindungsgegenstand ist dementsprechend die Verwendung von alkoxylierten Verbindungen, hergestellt durch Umsetzung von epoxidierten $C_{10-22}$-Carbonsäurederivaten mit $C_{2-4}$-Alkylenoxiden in Gegenwart von Alkoxylierungskatalysatoren sowie ein- und/oder mehrwertigen Alkoholen, zum Entfernen von Druckfarben aus Altpapieren und/oder Papierkreislaufwässern.

Die erfindungsgemäßen alkoxylierten Verbindungen werden Papierstoffsuspensionen vorzugweise in Mengen von 0,02 bis 2 Gew.-%, besonders bevorzugt von 0,1 bis 0,8 Gew.-%, jeweils bezogen auf lufttrockenen Papierstoff, zugesetzt. Lufttrockener Papierstoff bedeutet, daß sich im Papierstoff ein Gleichgewichtszustand an innerer Feuchte eingestellt hat. Dieser Gleichgewichtszustand hängt von der Temperatur und von der relativen Feuchte der Luft ab.

In vielen Fällen kann das Deinking-Ergebnis, d. h. die Entfernung von Druckfarben aus bedruckten Altpapieren gesteigert werden, wenn die erfindungsgemäßen alkoxylierten Verbindungen in Kombination mit beispielsweise $C_{10-22}$-Fettsäuren, wie Olinor[R]4010, Olinor[R]4020 und/oder Olinor[R]DG40 (Hersteller aller Produkte: Henkel KGaA), ethoxylierten Alkylalkohole mit 6 bis 22 C-Atomen, ethoxylierten Alkylphenolen, Polymeren, wie Polyacrylamiden und/oder Polydimethylaminoethylmethacrylat, und/oder Copolymeren, beschrieben beispielsweise in DE 38 39 479, eingesetzt werden. Die Gesamtmenge dieser fakultativen Bestandteile liegt zwischen 0,1 und 1 Gew.-%, bezogen auf lufttrockenen Papierstoff.

In Gegenwart der erfindungsgemäßen alkoxylierten Verbindungen lassen sich wasserverdünnbare und/oder lösungsmittelhaltige Druckfarben, beispielsweise Zeitungsrotationsfarben, Buchdruckfarben, Offsetdruckfarben, Illustrationstiefdruckfarben, Flexodruckfarben, Laserdruckfarben und/oder Verpackungstiefdruckfarben aus bedruckten Altpapieren, beispielsweise Zeitungen, Illustrierten, Computerpapieren, Zeitschriften, Broschüren, Formularen, Telefonbüchern und/oder Katalogen entfernen. Die in Gegenwart der erfindungsgemäßen alkoxylierten Verbindungen deinkten Altpapiere zeichnen sich durch sehr hohe Weißgrade aus.

Bedruckte Altpapiere werden bei einer Stoffdichte beispielsweise zwischen 1 und 5 Gew.-% in einem Stofflöser in wäßriger Lösung, die vorzugsweise 0 bis 1,5 Gew.-% 100%iges Wasserstoffperoxid, 0 bis 2,5 Gew.-% 99gew.-%ige NaOH, 0 bis 4,0 Gew.-% Natronwasserglas, Feststoffgehalt 35 Gew.-% (37 bis 40 °Be), 0,02 bis 2 Gew.-% erfindungsgemäße alkoxylierte Verbindungen und 0 bis 1 Gew.-% der oben genannten fakultativen Bestandteile - alle Gewichtsprozentangaben beziehen sich auf lufttrockenes Altpapier - enthält, bei Temperaturen zwischen 20 und 60 °C zerkleinert. Danach werden die Papierstoffsuspensionen in Wasser eingerührt oder mit Wasser versetzt, so daß 0,6 bis 1,6 gew.-%ige Papierstoffsuspensionen erhalten werden. Nach einer Verweilzeit zwischen 60 und 120 Minuten bei Temperaturen zwischen 20 und 60 °C werden die abgelösten Druckfarbenteilchen in an sich bekannter Weise durch Auswaschen oder durch Flotation aus den Papierstoffsuspensionen ausgeschieden (Ullmanns Enzyklopädie der technischen

EP 0 512 035 B1

Chemie, 4. Auflage, Band 17, Seiten 570 bis 571 (1979)). Vorzugsweise wird in an sich bekannter Weise, beispielsweise in einer Denver-Flotationszelle flotiert.

Bei Einsatz der erfindungsgemäßen alkoxylierten Verbindungen werden Druckfarben sowohl aus dem Altpapier als auch aus dem Kreislaufwasser entfernt. Die erfindungsgemäßen Verbindungen können jedoch auch zur separaten Reinigung von Papierkreislaufwässern eingesetzt werden. In diesen Fällen werden nach Zusatz von 2 bis 10 mg erfindungsgemäßer alkoxylierter Verbindungen pro Liter Kreislaufwasser die Druckfarbenteilchen beispielsweise durch Filtration oder Flotation ausgeschieden.

**Beispiele**

Beispiel 1

156 g epoxidiertes Sojaöl (ungefähre Fettsäurezusammensetzung: 25 Gew.-% Ölsäure, 48 Gew.-% Linolsäure, 7 Gew.-% Linolensäure, 2 Gew.-% Stearinsäure; Epoxidgehalt = 6,3 Gew.-%; Jodzahl = 4; Säurezahl = 0,2), 4 g Glycerin und 0,8 g 30 gew.-%ige methanolische Natriummethylat-Lösung wurden in einen Autoklaven gegeben. Nach Entfernen des Methanols im Vakuum wurde die Reaktionsmischung auf 180 °C aufgeheizt. Danach wurden 240 g Ethylenoxid zugegeben, so daß der Druck im Reaktor einen Wert von $8 \cdot 10^5$ Pa nicht überstieg. Nach 9 Stunden wurde zur Entfernung von noch vorhandenem Ethylenoxid evakuiert und auf Raumtemperatur (20 °C) abgekühlt. Das erhaltene flüssige Produkt hatte eine OH-Zahl (OHZ) von 120, eine Verseifungszahl (VZ) von 74,4 und einen Epoxidgehalt von 0,81 Gew.-%.

Beispiel 2

Analog Beispiel 1 wurden 300 g epoxidiertes Sojaöl (Kenndaten wie in Beispiel 1 angegeben) in Gegenwart von 20 g Glycerin und 2,1 g 30 gew.%iger methanolischer Natriummethylat-Lösung mit 700 g Ethylenoxid innerhalb von 6 Stunden bei 180 °C umgesetzt. Das erhaltene flüssige Produkt hatte eine OHZ von 86, eine VZ von 51 und einen Epoxidgehalt von 0,29 Gew.-%.

Beispiel 3

Analog Beispiel 1 wurden 390 g epoxidierter Sojafettsäure-2-ethylhexylester (Fettsäurezusammensetzung: wie in Beispiel 1 angegeben; Epoxidgehalt = 4,99 Gew.-%; VZ = 140,1) in Gegenwart von 20 g Glycerin und 2,1 g 30 gew.-%iger Natriummethylat-Lösung mit 610 g Ethylenoxid innerhalb von 10 Stunden umgesetzt. Das erhaltenen flüssige Produkt hatte eine OHZ von 89, eine VZ von 53 und einen Epoxidgehalt von 0,5 Gew.-%.

Anwendungsbeispiele

98 g lufttrockenes (= 90 g atro bei 8 % Feuchte; atro gleich absolut trocken) bedrucktes Altpapier, bestehend aus 50 Gew.-% Tageszeitungen und 50 Gew.-% Illustrierten, wurden bei 3,5 Gew.-% Stoffdichte im Laborpulper mit wäßriger Lösung, enthaltend 2,0 Gew.-% Natronwasserglas, Feststoffgehalt: 35 Gew.-% (37-40 ° Bé), 0,7 Gew.-% Wasserstoffperoxid, 100 gew.-%ig, 1,0 Gew.-% Natriumhydroxid, 99 gew.-%ig und 0,15 Gew.-% erfindungsgemäße alkoxylierte Verbindung (alle Gew.-%-Angaben bezogen auf lufttrockenen Papierstoff) mittels Dispergierscheibe bei 45 °C 10 Minuten zerkleinert. Danach wurde der Papierbrei mit Wasser auf 1 Gew.-% Stoffdichte verdünnt und 1 $^3/_4$ Stunden bei 45 °C stehen gelassen. Anschließend wurde 12 Minuten bei 45 °C in einer Denver-Laborflotationszelle bei 1900 Umdrehungen pro Minute flotiert. Nach der Flotation wurde der jeweilige Papierbrei auf einer Filternutsche von Wasser (Kreislaufwasser) getrennt und zwischen zwei Filterpapieren auf einer Fototrockenpresse zu einem Blatt geformt und bei 100 °C 90 Minuten getrocknet.

Die Deinking-Ergebnisse sind in Tabelle 1 zusammengefaßt. Die Deinkbarkeitsmaßzahl (DEM) wurde aus den Reflektionsfaktoren $R_{457nm}$ (Weißgrad) der bedruckten (BS), deinkten (DS) und unbedruckten (US) Papierstoffe nach folgender Formel berechnet:

$$DEM\ (\%) = \frac{Wei\beta grad\ (DS) - Wei\beta grad\ (BS)}{Wei\beta grad\ (US) - Wei\beta grad\ (BS)} \times 100$$

(0 % bedeutet keine Druckfarbenentfernung, 100 % bedeutet quantitative Druckfarbenentfernung). Das Kreislaufwasser war in allen Fällen klar.

Tabelle 1

| eingesetzte alkoxylierte Verbindungen, hergestellt nach Beispiel | $R_{457}{}^{1)}$ (US) | $R_{457}$ (BS) | $R_{457}$ (DS) | DEM (%) |
|---|---|---|---|---|
| 1 | 62 | 41 | 59 | 84 |
| 2 | 62 | 41 | 59 | 85 |
| 3 | 62 | 41 | 60 | 86 |

[1)] $R_{457}$ bedeutet $R_{457nm}$

**Patentansprüche**

1. Alkoxylierte Verbindungen, hergestellt durch Umsetzung von epoxidierten $C_{10-22}$-Carbonsäurederivaten mit $C_{2-4}$-Alkylenoxiden in Gegenwart von Alkoxylierungskatalysatoren sowie ein- und/oder mehrwertigen Alkoholen.

2. Alkoxylierte Verbindungen nach Anspruch 1, dadurch gekennzeichnet, daß diese einen Alkylenoxidgehalt zwischen 20 und 90 Gew.-%, vorzugsweise zwischen 40 und 80 Gew.-% haben.

3. Alkoxylierte Verbindungen nach einem oder beiden der Ansprüche 1 bis 2, dadurch gekennzeichnet, daß $C_{2-4}$-Alkyienoxide Ethylenoxid und/oder Propylenoxid sind.

4. Alkoxylierte Verbindungen nach einem oder mehreren der Ansprüche 1 bis 3, dadurch gekennzeichnet, daß diese in Gegenwart von ein- und/oder mehrwertigen Alkoholen in Mengen von 0,1 bis 2,0, vorzugsweise von 0,2 bis 1,0 OH-Gruppen pro Epoxidgruppe hergestellt werden.

5. Alkoxylierte Verbindungen nach einem oder mehreren der Ansprüche 1 bis 4, dadurch gekennzeichnet, daß epoxidierte Carbonsäurederivate in der Kohlenstoffkette der Carbonsäurereste 16 bis 22 C-Atome haben.

6. Alkoxylierte Verbindungen nach einem oder mehreren der Ansprüche 1 bis 5, dadurch gekennzeichnet, daß epoxidierte Carbonsäurederivate epoxidierte Mono-, Di- und/oder Triglyceride und/oder epoxidierte Carbonsäurealkylester mit 1 bis 18 C-Atomen im einwertigen Alkoholrest sind.

7. Verfahren zur Herstellung von alkoxylierten Verbindungen, dadurch gekennzeichnet, daß epoxidierte $C_{10-22}$-Carbonsäurederivate mit $C_{2-4}$-Alkylenoxiden in Gegenwart von Alkoxylierungskatalysatoren sowie ein- und/oder mehrwertigen Alkoholen bei Temperaturen zwischen 110 und 220 °C und Drücken zwischen $10^5$ und $2 \cdot 10^6$ Pa umgesetzt werden.

8. Verfahren nach Anspruch 7, dadurch gekennzeichnet, daß die Umsetzungen bei Temperaturen zwischen 150 und 190 °C und Drücken zwischen $3 \cdot 10^5$ und $9 \cdot 10^5$ Pa durchgeführt werden.

9. Verfahren nach einem oder beiden der Ansprüche 7 bis 8, dadurch gekennzeichnet, daß Alkylenoxide in solchen Mengen eingesetzt werden, daß der Alkylenoxidgehalt der alkoxylierten Verbindungen zwischen 20 und 90 Gew.-%, vorzugsweise zwischen 40 und 80 Gew.-% liegt.

**10.** Verfahren nach einem oder mehreren der Ansprüche 7 bis 8, dadurch gekennzeichnet, daß die Umsetzungen mit Ethylenoxid und/oder Propylenoxid durchgeführt werden.

**11.** Verfahren nach einem oder mehreren der Ansprüche 7 bis 10, dadurch gekennzeichnet, daß die Umsetzungen in Gegenwart von ein- und/oder mehrwertigen Alkoholen in Mengen von 0,1 bis 2,0, vorzugsweise von 0,2 bis 1,0 OH-Gruppen pro Epoxidgruppe durchgeführt werden.

**12.** Verfahren nach einem oder mehreren der Ansprüche 7 bis 11, dadurch gekennzeichnet, daß epoxidierte Carbonsäurederivate mit 16 bis 22 Kohlenstoffatomen in den Carbonsäureresten eingesetzt werden.

**13.** Verfahren nach einem oder mehreren der Ansprüche 7 bis 12, dadurch gekennzeichnet, daß als Carbonsäurederivate epoxidierte Mono-, Di- und/oder Triglyceride und/oder epoxidierte Carbonsäurealkylester mit 1 bis 18 C-Atomen im einwertigen Alkoholrest eingesetzt werden.

**14.** Verwendung von alkoxylierten Verbindungen, hergestellt durch Umsetzung von epoxidierten $C_{10-22}$-Carbonsäurederivaten mit $C_{2-4}$-Alkylenoxiden in Gegenwart von Alkoxylierungskatalysatoren sowie ein- und/oder mehrwertigen Alkoholen, zum Entfernen von Druckfarben aus Altpapieren und/oder Papierkreislaufwässern.

**Claims**

**1.** Alkoxylated compounds prepared by reaction of epoxidized $C_{10-22}$ carboxylic acid derivatives with $C_{2-4}$ alkyleneoxides in the presence of alkoxylation catalysts and mono- and/or polyhydric alcohols.

**2.** Alkoxylated compounds as claimed in claim 1, characterized in that they have an alkylene oxide content of 20 to 90% by weight and preferably 40 to 80% by weight.

**3.** Alkoxylated compounds as claimed in one or both of claims 1 and 2, characterized in that the $C_{2-4}$ alkylene oxides are ethylene oxide and/or propylene oxide.

**4.** Alkoxylated compounds as claimed in one or more of claims 1 to 3, characterized in that they are prepared in the presence of mono- and/or polyhydric alcohols in quantities of 0.1 to 2.0 and preferably 0.2 to 1.0 OH groups per epoxide group.

**5.** Alkoxylated compounds as claimed in one or more of claims 1 to 4, characterized in that the epoxidized carboxylic acid derivatives contain 16 to 22 carbon atoms in the carbon chain of the carboxylic acid residues.

**6.** Alkoxylated compounds as claimed in one or more of claims 1 to 5, characterized in that the epoxidized carboxylic acid derivatives are epoxidized mono-, di- and/or triglycerides and/or epoxidized carboxylic acid alkyl esters containing 1 to 18 carbon atoms in the monohydric alcohol function.

**7.** A process for the production of alkoxylated compounds, characterized in that epoxidized $C_{10-22}$ carboxylic acid derivatives are reacted with $C_{2-4}$ alkylene oxides in the presence of alkoxylation catalysts and mono- and/or polyhydric alcohols at temperatures in the range from 110 to 220°C and under pressures of $10^5$ to $2 \cdot 10^6$ Pa.

**8.** A process as claimed in claim 7, characterized in that the reactions are carried out at temperatures of 150 to 190°C under pressures of $3 \cdot 10^5$ to $9 \cdot 10^5$ Pa.

**9.** A process as claimed in one or both of claims 7 and 8, characterized in that the alkylene oxides are used in such quantities that the alkylene oxide content of the alkoxylated compounds is between 20 and 90% by weight and preferably between 40 and 80% by weight.

**10.** A process as claimed in one or more of claims 7 to 8, characterized in that the reactions are carried out with ethylene oxide and/or propylene oxide.

**11.** A process as claimed in one or more of claims 7 to 10, characterized in that the reactions are carried out in the presence of mono- and/or polyhydric alcohols in quantities of 0.1 to 2.0 and preferably 0.2 to 1.0 OH groups per epoxide group.

**12.** A process as claimed in one or more of claims 7 to 11 characterized in that epoxidized carboxylic acid derivatives containing 16 to 22 carbon atoms in the carboxylic acid residues are used.

**13.** A process as claimed in one or more of claims 7 to 12, characterized in that epoxidized mono-, di- and/or triglycerides and/or epoxidized carboxylic acid alkyl esters containing 1 to 18 carbon atoms in the monohydric alcohol function are used as the carboxylic acid derivatives.

**14.** The use of alkoxylated compounds prepared by reaction of epoxidized $C_{10-22}$ carboxylic acid derivatives with $C_{2-4}$ alkylene oxides in the presence of alkoxylation catalysts and mono- and/or polyhydric alcohols for the removal of printing inks from wastepaper and/or paper circuit waters.

**Revendications**

**1.** Composés alcoxylés, obtenus par réaction de dérivés d'acide carboxylique en $C_{10}$ à $C_{22}$ époxydés avec des oxydes d'alcoylène en $C_2$-$C_4$ en présence de catalyseurs d'alcoxylation ainsi que d'alcools uni- et/ou plurivalents.

**2.** Composés alcoxylés selon la revendication 1, caractérisés en ce que ceux-ci possèdent une teneur en oxyde d'alcoylène comprise entre 20 et 40 % en poids, de préférence entre 40 et 80 % en poids.

**3.** Composés alcoxylés selon l'une ou les deux revendications 1 à 2, caractérisés en ce que les oxydes d'alcoylène en $C_2$-$C_4$ sont l'oxyde d'éthylène et/ou l'oxyde de propylène.

**4.** Composés alcoxylés selon l'une ou plusieurs des revendications 1 à 3, caractérisés en ce que ceux-ci sont obtenus en présence d'alcools uni- et/ou plurivalents en quantités allant de 0,1 à 2,0 de préférence de 0,2 à 1,0 groupe OH par groupe époxyde.

**5.** Composés alcoxylés selon l'une ou plusieurs des revendications 1 à 4, caractérisés en ce que les dérivés d'acides carboxyliques époxydés ont dans la chaîne carbonée des radicaux d'acide carboxylique de 16 à 22 atomes de C.

**6.** Composés alcoxylés selon l'une ou plusieurs des revendications 1 à 5, caractérisés en ce que les dérivés d'acides carboxyliques époxydés sont des mono-, diet/ou triglycérides et/ou des esters d'alcoyle d'acide carboxylique ayant de 1 à 18 atomes de carbone dans le radical alcoolique univalent.

**7.** Procédé d'obtention de composés alcoxylés, caractérisé en ce que des dérivés d'acide carboxylique en $C_{20}$-$C_{22}$ époxydés sont mis à réagir avec des oxydes d'alcoylène en $C_2$-$C_4$ en présence de catalyseurs d'alcoxylation ainsi que d'alcools uni- et/ou plurivalents à des températures comprises entre 110 et 220 °C et à des pressions entre $10^5$ et $2 \cdot 10^6$ Pa.

**8.** Procédé selon la revendication 7, caractérisé en ce que les réactions sont effectuées à des températures entre 150 et 190 °C et à des pressions entre $3.10^5$ et $9.10^5$ Pa.

**9.** Procédé selon l'une ou selon les deux revendications 7 à 8, caractérisé en ce que l'on met en oeuvre des oxydes d'alcoylène en quantités telles que la teneur en oxyde d'alcoylène, des composés alcoxylés se situe entre 20 et 90 % en poids, de préférence entre 40 et 80 % en poids.

**10.** Procédé selon l'une ou plusieurs des revendications 7 à 8, caractérisé en ce que les réactions sont effectuées avec de l'oxyde d'éthylène et/ou de l'oxyde de propylène.

**11.** Procédé selon l'une ou plusieurs des revendications 7 à 10, caractérisé en ce que les réactions sont effectuées en présence d'alcools uni- ou plurivalents en quantités allant de 0,1 à 2,0 - de préférence de 0,2 à 1,0 groupe OH par groupe époxy.

**12.** Procédé selon l'une ou plusieurs des revendications 7 à 11, caractérisé en ce que des dérivés d'acides carboxyliques époxydés ayant de 16 à 22 atomes de carbone dans les radicaux d'acide carboxylique sont mis en oeuvre.

**13.** Procédé selon l'une ou plusieurs des revendications 7 à 12, caractérisé en ce que comme dérivés d'acide carboxylique, on met en oeuvre des mono-, di- et/ou triglycérides époxydés et/ou des esters d'alcoyle d'acide carboxylique époxydés ayant de 1 à 18 atomes de carbone dans le radical alcool univalent.

**14.** Utilisation des composés alcoxylés, produits par réaction de dérivés d'acide carboxylique en $C_{10}$-$C_{22}$ époxydés avec des oxydes d'alcoylène en $C_2$-$C_4$ en présence de catalyseurs d'alcoxylation ainsi que d'alcools uni- ou plurivalents en vue de l'élimination des couleurs d'impression des vieux papiers et/ou des eaux de recyclage du papier.